# EUROPÄISCHE PATENTSCHRIFT

(11) **EP 3 707 099 B1**
(45) Veröffentlichungstag und Bekanntmachung des Hinweises auf die Patenterteilung: **16.10.2024**
(21) Anmeldenummer: 18807853.9
(22) Anmeldetag: 30.10.2018
(51) Int. Cl.: C01B 33/027, C01B 33/029, B01J 13/00, B01J 19/00, B01J 19/10, H01M 4/38

(54) **VERFAHREN ZUR HERSTELLUNG VON HYDROGENIERTEN AMORPHEN SILICIUMHALTIGEN KOLLOIDEN UND/ODER KOMPOSIT-KOLLOIDEN UND ZUR VERKAPSELUNG VON SUBSTANZEN MIT HYDROGENIERTEN AMORPHEN SILICIUMHALTIGEN KOMPOSIT-KOLLOIDEN, SOWIE HYDROGENIERTE AMORPHE SILICIUMHALTIGE KOLLOIDE UND/ODER KOMPOSIT-KOLLOIDE UND MIT SILICIUMHALTIGEN KOMPOSIT-SCHICHTEN VERKAPSELTE SUBSTANZEN UND DEREN VERWENDUNG**
METHOD FOR PRODUCING HYDROGENATED AMORPHOUS SILICON-CONTAINING COLLOIDS AND/OR COMPOSITE COLLOIDS AND FOR ENCAPSULATING SUBSTANCES WITH HYDROGENATED AMORPHOUS SILICON-CONTAINING COMPOSITE COLLOIDS, HYDROGENATED AMORPHOUS SILICON-CONTAINING COLLOIDS AND/OR COMPOSITE COLLOIDS, SUBSTANCES ENCAPSULATED WITH SILICON-CONTAINING COMPOSITE LAYERS, AND USE THEREOF
PROCÉDÉ POUR FABRIQUER DES COLLOÏDES ET/OU DES COLLOÏDES COMPOSITES CONTENANT DU SILICIUM AMORPHE HYDROGÉNÉ ET POUR ENCAPSULER DES SUBSTANCES À L'AIDE DE COLLOÏDES COMPOSITES CONTENANT DU SILICIUM AMORPHE HYDROGÉNÉ, AINSI QUE DES COLLOÏDES ET/OU DES COLLOÏDES COMPOSITES CONTENANT DU SILICIUM AMORPHE HYDROGÉNÉ ET SUBSTANCES ENCAPSULÉES AVEC DES COUCHES COMPOSITES CONTENANT DU SILICIUM ET LEUR UTILISATION

(30) Priorität: 07.11.2017 DE 102017010263
(43) Veröffentlichungstag der Anmeldung: 16.09.2020
(73) Patentinhaber: Forschungszentrum Jülich GmbH, 52425 Jülich (DE)
(72) Erfinder: CADIZ BEDINI, Andrew Paolo, 52062 Aachen (DE); KLINGEBIEL, Benjamin, 52441 Linnich (DE); HAAS, Stefan, 52499 Baesweiler (DE); FLOHRE, Jan, 52074 Aachen (DE); CARIUS, Reinhard, 52428 Jülich (DE); CHEN, Chunguang, 52428 Jülich (DE); NOTTEN, Peter, 5583 XV Waalre (NL)
(86) Internationale Anmeldenummer: PCT/DE2018/000320
(87) Internationale Veröffentlichungsnummer: WO 2019/091506

(56) Entgegenhaltungen:
- EP-A1- 1 867 993
- EP-A1- 3 026 736
- WO-A1-2015/085980
- APC BEDINI, S MUTHMANN, J ALLGAIER, K BITTKAU, F. FINGER, R. CARIUS: "Liquid hydridosilane precursor prepared from cyclopentasilane via sonication at low temperatures without the action of light", ULTRASONICS SONOCHEMISTRY, vol. 34, 26 May 2016 (2016-05-26), pages 289 - 293, XP002788819, DOI: 10.1016/j.ultsonch.2016.05.039

## Beschreibung

Die Erfindung betrifft ein Verfahren zur Herstellung von hydrogenierten amorphen siliciumhaltigen Kolloiden oder Komposit-Kolloiden und zur Verkapselung von Substanzen mit hydrogenierten amorphen siliciumhaltigen Komposit-Schichten, sowie hydrogenierte amorphe siliciumhaltige Kolloide oder Komposit-Kolloide und mit siliciumhaltigen Komposit-Schichten verkapselte Substanzen und deren Verwendung. Die Erfindung betrifft weiterhin hydrogenierte amorphe siliciumhaltige Kolloide oder Komposit-Kolloide, die hohl sind und eine siliciumhaltige Schale aufweisen.

Nach dem Stand der Technik werden gasförmige, niedere Silane, d.h. Silicium-WasserstoffVerbindungen, wie Monosilan (SiH₄) und Disilan (Si₂H₆) als Precursoren verwendet um siliciumhaltige Nanopartikel mittels Vakuumprozessen wie z.B. PECVD (Plasma-Enhanced Chemical Vapor Deposition) Hot-Wire-CVD oder Hot-Wall Reaktoren herzustellen.

Mit Silanen, die flüssig vorliegen, wie z.B. Trisilan Si₃H₈ sind der Anmelderin keine Verfahren nach dem Stand der Technik bekannt, bei denen bei Normaldruck und Normaltemperatur gearbeitet wird.

Ein Verfahren, wie es in L.E. Pell, et.al., "Synthesis of amorphous silicon colloids by trisilane thermolysis in high temperature supercritical solvents", Langmuir 20 (2004) 6546-6548 beschrieben wird, offenbart eine Methode bei der amorphe und wasserstoffhaltige Kolloide in einem Autoklaven bei Temperaturen zwischen 400 °C und 500 °C und Drücken zwischen 200 bar und 400 bar hergestellt werden.

Für Cyclohexasilan (Si₆H₁₂) ist ein Gasphasen-Pyrolyse-Verfahren bekannt, das die Herstellung von amorphen und wasserstoffhaltigen Nanopartikeln ermöglicht, welches jedoch bei Temperaturen zwischen 900 °C und 1100 °C durchgeführt wird. Das Verfahren ist in der Veröffentlichung "Synthesis of silicon quantum dots using cyclohexasilane (Si6H12)", Guruvenket et al., J. Mater. Chem. C, 2016, 4, 8206 beschrieben.

Den Erfindern sind keine Verfahren bekannt, die es ermöglichen, basierend auf flüssigen silicium-wasserstoffhaltigen Verbindungen und akustischer Kavitation, Kolloide oder Komposit-Kolloide bei Raumtemperatur und Atmosphärendruck herzustellen. Den Erfindern sind weiterhin keine hydrogenierten siliciumhaltigen Komposit-Kolloide bekannt, die ohne weitere chemische Behandlungsschritte hohl und hydrogeniert sind und eine siliciumhaltige Schale aufweisen.

Mit den Verfahren nach dem Stand der Technik sind verschiedene Nachteile verbunden. Meist führen die Verfahren nach dem Stand der Technik zu kristallinen oder nicht hydrogenierten Nanopartikeln. Die Verfahren sind technisch aufwändig und beruhen auf Gasphasen-Hochtemperatur- oder Plasma- oder Laser-Technik-Verfahren oder benötigen Hochdruck. Diese Nachteile des Stands der Technik haben beispielsweise zur Folge, dass eine direkte Verkapselung von Stoffen bzw. Fremdsubstanzen mit hydrogenierten amorphen siliciumhaltigen Beschichtungen nicht möglich ist.

Aus der WO2015085980 ist ein Verfahren zur Polymerisation einer Zusammensetzung enthaltend Hydridosilane und die anschließende Verwendung der Polymerisate zur Herstellung von siliziumhaltigen Schichten bekannt, bei dem ein Substrat bereitgestellt wird, eine Zusammensetzung bereitgestellt wird, enthaltend wenigstens ein Hydridosilan, das entweder in mindestens einem organischen und/oder anorganischen Lösungsmittel gelöst ist oder enthaltend wenigstens ein Hydridosilan, das auch ohne Lösungsmittel bereits flüssig vorliegt, wobei die zuvor genannten Hydridosilane wenigstens ein lineares und/oder verzweigtes Hydridosilan der allgemeinen Formel SiH mit n 2 3 und/oder ein zyklisches Hydridosilan der allgemeinen Formel SiH mit n 2 3 umfassen und einer Polymerisation der zuvor genannten Zusammensetzung mittels akustischer Kavitation durchgeführt wird.

Aus der Veröffentlichung "Liquid hydridosilane precursor prepared from cyclopentasilane via sonication at low temperatures without the action of light" von APC Bedini, S. Muthmann, J. Allgaier. K. Bittkau, F. Finger und R. Carius; Ultrasonics Sonochemistry, Bd.34, 26. Mai 2016, Seiten 289-293 wird ein Verfahren zur Herstellung einer Tinte offenbart, bei der die Tinte aus flüssigen Hydridosilanen besteht und bei dem eine Vorläuferlösung bestehend aus Cyclopentasilan in einer N₂-Atmosphäre mit Ultraschall bei 75°C beschickt wird, so dass eine Ringöffnungspolymerisation des Cyclopentasilans stattfindet.

Aus der EP3026736 sind amorphe, hydrogenierte Siliciumpartikel bekannt, die als Anodedenmaterial eingesetzt werden.

Aus der EP1867993 sind metallische Nanopartikel bekannt, die mit einer amorphen siliciumhaltigen Schale beschichtet sind, die für biomedizinsiche Zwecke verwendet werden.

Aus dem Stand der Technik sind zwar amorphe, hydrogenierte Siliciumpartikel oder Nanopartikel mit einer amorphen siliciumhaltigen Schale bekannt. Jedoch sind keine Kompositkolloide und deren Verwendung bekannt, die sowohl eine siliciumhaltigen Schale aufweisen, eine sphärische Geometrie aufweisen und einen sphärischen Hohlraum aufweisen. Auch mit den nach dem Stand der Technik bekannten Verfahren können keine derartigen Komposit-kolloide hergestellt werden.

Es ist daher die Aufgabe der Erfindung ein Verfahren zur Herstellung von hydrogenierten amorphen siliciumhaltigen Komposit-Kolloiden, die eine siliciumhaltigen Schale aufweisen, eine sphärische Geometrie aufweisen und einen sphärischen Hohlraum aufweisen, bereitzustellen und ein Verfahren zur Verkapselung von Substanzen mit diesen hydrogenierten amorphen siliciumhaltigen Komposit-Kolloiden bereitzustellen, sowie diese hydrogenierten amorphen siliciumhaltigen Komposit-Kolloide und deren Verwendung zur Verfügung zu stellen.. Insbesondere soll das Verfahren bei niedrigen Temperaturen, wie beispielsweise Raumtemperatur und niedrigeren Drücken, wie beispielsweise Atmosphärendruck durchgeführt werden können. Das Verfahren soll einfach durchführbar sein und wenig apparativen Aufwand erfordern. Das Verfahren soll auch die Verkapselung oder Einbettung von Additiven oder Substanzen mit diesen hydrogenierten amorphen siliciumhaltigen Komposit-Kolloiden ermöglichen oder eine Beschichtung dieser mit hydrogenierten amorphen siliciumhaltigen Komposit-Kolloiden ermöglichen. Weiterhin sollen hydrogenierte amorphe siliciumhaltige Komposit-Kolloide, sowie Substanzen zur Verfügung gestellt werden, welche mit hydrogenierten, amorphen, siliciumhaltigen Beschichtungen verkapselt sind.

Im Rahmen der vorliegenden Erfindung werden unter der Bezeichnung "Kolloid" sowohl flüssige als auch feste Nanopartikel als auch flüssige oder feste Partikel verstanden, die nicht nanoskalige Maße aufweisen, wie beispielsweise Mikropartikel oder Agglomerate. Die Kolloide können Emulsionen oder Suspensionen von Tröpfchen oder Gasen in einer Flüssigkeit sein, die während oder nach der erfindungsgemäßen Beschallung/Kavitation zu festen Kolloiden umgewandelt werden.

Im Rahmen der vorliegenden Anmeldung werden unter der Bezeichnung "Komposit-Kolloid" Kolloide verstanden, die mehr als nur aus Silicium bestehen, wie beispielsweise Kolloide bei denen beispielsweise ein Kohlenstoff oder ein Medikament mitsynthetisiert und/oder eingebettet und/oder verkapselt wurde.

Im Rahmen der vorliegenden Anmeldung wird unter der Bezeichnung "Verkapselung" allgemein eine Beschichtung oder Einbettung einer Substanz mit oder in ein Kolloid verstanden.

Ausgehend vom Oberbegriff des Anspruchs 1 und der nebengeordneten Ansprüche wird die Aufgabe erfindungsgemäß gelöst mit den im kennzeichnenden Teil des Anspruchs 1 und der nebengeordneten Ansprüche angegebenen Merkmalen.

Mit dem erfindungsgemäßen Verfahren ist es nunmehr möglich, ein Verfahren zur Herstellung von hydrogenierten amorphen siliciumhaltigen Koloiden oder Komposit-Kolloiden und zur Verkapselung von Substanzen mit hydrogenierten amorphen siliciumhaltigen kolloiden oder Komposit-Kolloiden, sowie hydrogenierte, amorphe, siliciumhaltige Kolloide oder Komposit-Kolloide und mit siliciumhaltigen Kolloiden oder Komposit-Kolloiden verkapselte Substanzen und deren Verwendung zur Verfügung zu stellen, welches die o.g. Nachteile meidet. Insbesondere, kann das Verfahren bei niedrigen Temperaturen, wie beispielsweise Raumtemperatur und niedrigeren Drücken, wie beispielsweise Atmosphärendruck durchgeführt werden. Das Verfahren ist einfach durchführbar und erfordert wenig apparativen Aufwand. Das Verfahren ermöglicht auch die Verkapselung von Stoffen mit hydrogenierten amorphen siliciumhaltigen Kolloiden oder Komposit-Kolloiden oder mit hydrogenierten amorphen siliciumhaltigen Beschichtungen. Weiterhin können hydrogenierte amorphe siliciumhaltige Komposit-Kolloide, sowie Substanzen zur Verfügung gestellt werden, welche mit hydrogenierten, amorphen, siliciumhaltigen Beschichtungen verkapselt sind. Das Verfahren ermöglicht die Herstellung von hydrogenierten amorphen siliciumhaltigen Kolloiden und Komposit-Kolloiden, die eine siliciumhaltige Schale aufweisen, eine sphärische Geometrie aufweisen und einen sphärischen Hohlraum aufweisen.

Die nach dem erfindungsgemäßen Verfahren hergestellten Kolloide und Komposit-Kolloide weisen zusätzliche vorteilhafte Eigenschaften auf.

Die erfindungsgemäßen siliciumhaltigen Kolloide oder Komposit -Kolloide haben bevorzugt eine sphärische Geometrie. Eine siliciumhaltige oder auch eine hydrogenierte siliciumhaltige Schale umgibt den Hohlraum der Kolloide oder Koposit-Kolloide. Die Kolloide oder Komposit -Kolloide haben vorzugsweise einen Durchmesser zwischen 50 bis 200 nm. Die siliciumhaltige Schale hat vorzugsweise eine Dicke im Bereich von 3 bis 10 nm.

Beispielsweise ist nicht nur das Volumen aber auch die äußere Oberfläche mit verschieden wasserstoffhaltigen Gruppen terminiert, nämlich mit -SiH, -SiH₂ und -SiH₃ Gruppen. Darüber hinaus, da sich die Oberfläche in einem nichtoxidierten Zustand (mit Sauerstoff) befindet, erleichtern die besonderen Eigenschaften der Oberflächen erheblich den Aufwand der Funktionalisierung der Oberflächenchemie der Kolloide bzw. Komposit-Kolloide.

Eine weitere vorteilhafte Eigenschaft der Kolloide bzw. Komposit-Kolloide, ist die erfindungsgemäße nano- bzw. mesoporöse Anordnung, die sie als dünne Schichten auf festen Substraten aufweisen. Gemäß Anwendung können solche Schichten mittels Rotations-, Drop-, Sprüh-, Doktorblade- oder Rackelbeschichtung hergestellt werden. Ohne keinerlei Zugabe von Binde- oder Leitfähigkeitsmitteln weisen diese erfindungsgemäß hergestellten porösen Schichten überraschend hohe spezifische Kapazitäten und lange Stabilitäten bei der Lithiierung in Lithium-Ionen-Halb-Zellen aus und sind damit überwiegend vorteilhaft gegenüber dem Stand der Technik. Vorteilhafte Weiterbildungen der Erfindung sind in den Unteransprüchen angegeben.

Im Folgenden wird die Erfindung in ihrer allgemeinen Form beschrieben, ohne dass dies einschränkend auszulegen ist.

Gemäß dem erfindungsgemäßen Verfahren, wird ein Hydridosilan oder ein Hydrodosilan-Derivat oder eine Mischung von verschiedenen Hydridosilanen und/oder Hydridosilan-Derivaten, die entweder in mindestens einem organischen und/oder anorganischen Lösungsmittel gelöst sind oder wenigstens ein Hydridosilan oder ein Hydrodosilan-Derivat oder eine Mischung von verschiedenen Hydridosilanen und/oder Hydridosilan-Derivaten, die ohne Lösungsmittel bereits flüssig vorliegen mit Kavitation beaufschlagt. Die Kavitation kann beispielsweise durch eine magnetostriktive oder piezoelektrische Ultraschallquelle und/oder durch einen flüssigkeitsbetriebenen oder gasbetriebenen Schallwandler erzeugt werden.

Dabei entstehen erfindungsgemäß hydrogenierte, amorphe, siliciumhaltige Komposit-Kolloide. Unter Kolloid sollen im Rahmen der vorliegenden Erfindung beispielsweise auch Festkörper oder Partikel oder Teilchen oder Aggregate oder Agglomerate verstanden werden, die in mindestens einer Längendimension eine Größe zwischen 0,1 nm und 100 µm aufweisen.

Als Hydridosilane können erfindungsgemäß folgende Verbindungen eingesetzt werden: lineare oder verzweigte Siliciumwasserstoffe der Summenformel SiₙH₂ₙ₊ₙ, mit n = 1, 2, 3, ..., oder cyclische Siliciumwasserstoffe der Form SiₙH₂ₙ, mit n = 5, 6, ....

So können beispielsweise als Hydridosilane mindestens eine Komponente aus der Gruppe, bestehend aus linearen oder verzweigten Silanen mit der Summenformel SiₙH₂ₙ+₂ (mit n ≥ 2) oder zyklischen Silanen mit der Summenformel SiₙH₂ₙ (mit n > 4) oder aus Organosilanen, Halogensilanen oder Organohalogensilanen, vorzugsweise aus der Gruppe Trisilan, Tetrasilan, Pentasilan, Hexasilan, Heptasilan, Cyclopentasilan, Cyclohexasilan, Neopentasilan, Trichlorsilan, oder auch flüssige Germane (GeₙH₂ₙ+₂, mit n ≥ 2) eingesetzt werden.

Als Hydridosilan-Derivate können erfindungsgemäß folgende Verbindungen eingesetzt werden: Verbindungen mit allgemeiner Formel SiHₙX₄₋ₙ (mit X = F, Cl, Br, I, CH₃ und 1 < n < 4).

Unter Mischlösung sind Lösungen zu verstehen, enthaltend Mischungen aus verschiedenen Hydridosilanen oder aus verschiedenen Hydridosilan-Derivaten oder eine Kombination von Hydridosilanen und Hydridosilan-Derivaten.

Es können auch mindestens zwei Hydridosilane eingesetzt werden, so dass das durch das Verfahren hergestellte Komposit-Kolloid eine Unterkombination aus mindestens zwei Komponenten, insbesondere der genannten Hydridosilane ist.

Bei dem Verfahren implodieren durch die Kavitation erzeugten Mikrobläschen in der Lösung.

Als Lösungsmittel sind sowohl organische als auch anorganische Lösungsmittel geeignet. Als Lösungsmittel können beispielsweise lineare (n) und iso (i) Alkane wie n- oder i-Pentan (C₆H₁₂), n- oder i-Hexan (C₆H₁₄), sowie zyklische Alkane wie Cyclohexan (C₆H₁₂), Cycloheptan (C₇H₁₄), Cyclooctan (CₐH₁₆), oder auch aromatische Verbindungen wie Benzol, Toluol, Xylol, Naphthalin eingesetzt werden.

Als Lösungsmittel können auch sauerstoffhaltige oder anorganische Verbindungen wie Wasser, Alkohole, Ether, Alkoxyalkane, Siloxane eingesetzt werden.

Aber auch alle gemäß Stand der Technik weiteren geeigneten und dem Fachmann bekannten organischen Lösungsmittel, insbesondere hochsiedende Lösungsmittel wie höhere Alkane oder Terpene, wie beispielsweise n-Dodecan oder Squalen, können eingesetzt werden. Als Lösungsmittel kann auch ein Lösungsmittelgemisch aus mindestens zwei Komponenten, insbesondere der zuvor genannten Lösungsmittel eingesetzt werden.

Das Verfahren kann in einem Temperaturbereich zwischen -120 °C und 250 °C durchgeführt werden. Bevorzugt ist ein Temperaturbereich zwischen -30 °C und 125 °C. Insbesondere kann das Verfahren bei Umgebungstemperatur, insbesondere Raumtemperatur durchgeführt werden.

Das Verfahren kann in einem Druckbereich zwischen 0,5 bar und 1000 bar durchgeführt werden. Bevorzugt ist ein Druckbereich zwischen 1 bar und 100 bar. Insbesondere kann das Verfahren bei Normaldruck, insbesondere Atmosphärendruck durchgeführt werden.

Der Ultraschall kann beispielsweise in einem Frequenzbereich von 15 kHz - 50 MHz liegen, ist aber nicht auf diesen Bereich beschränkt.

In einer weiteren Ausgestaltung der Erfindung kann die Lösung auch Additive enthalten. Unter der Bezeichnung "Additive" sollen im Rahmen der vorliegenden Erfindung Substanzen verstanden werden, die beim Einsetzen in einer Lösung vor, während, oder nach der Beschallung, zu keinen und/oder schwachen und/oder starken chemischen Bindungen mit dem Wasserstoff und/oder dem Silicium der erzeugten Kolloide führen.

Additive, die zu chemischen Bindungen führen, können feste, flüssige oder gasförmige Substanzen sein, wie beispielsweise lithiumhaltige Verbindungen oder Dotierstoffe wie borhaltige und/oder phosphorhaltige Verbindungen oder Verbindungen aller Art, die zu entsprechenden Zusammensetzungen, Legierungen oder Komposit-Kolloiden überführen. Unter dem Begriff "Komposit-Kolloid" soll verstanden werden, Kolloide, die durch das erfindungsgemäße Verfahren nicht nur aus Wasserstoff und Silicium bestehen.

Additive, die keine und/oder nur schwache chemische Bindungen mit dem Wasserstoff und/oder mit dem Silicium der erzeugten Kolloide eingehen sollen, können feste, flüssige oder gasförmige Moleküle sein, wie beispielsweise Nanopartikel oder Therapeutika.

Diese Additive werden auch "zu verkapselnden" oder "zu beschichtenden" Substanzen genannt. Befinden sich diese Substanzen innerhalb und/oder auf der Oberfläche des erzeugten Kolloids, wird dieser Prozess im Rahmen der vorliegenden Erfindung als eine "Einbettung" oder "Verkapselung" oder aber auch als "Beschichtung" verstanden. Im Unterschied zur Verkapselung ist es bei der Beschichtung möglich, dass die Beschichtung eine unvollkommene Verkapselung darstellt, bei der sich die Substanzen nicht zu 100% innerhalb und/oder auf der Oberfläche des erzeugten Kolloids befinden. Erfindungsgemäß soll die ursprüngliche Zielwirkung dieser Substanzen teilweise oder vollkommen erhalten bleiben. Die Substanzen, die eingebettet oder verkapselt werden sollen, sollten bevorzugt so klein sein, dass sie in die Bläschen die durch die Kavitation erzeugt werden, hineinpassen. Sie sollten daher vorteilhaft einen Durchmesser von < 100 µm aufweisen.

Bei einer Beschichtung können die Substanzen auch einen größeren Durchmesser von > 100 µm aufweisen, da diese Substanzen nach und nach auf der Oberfläche der Komposit-Kolloide beschichtet werden, in dem sie bei der Beschallung bzw. bei der Implosion der Kavitationsbläschen in der Nähe dieser Bläschen zugegeben werden. Die Additive oder Substanzen haben zur Folge dass, siliciumhaltige Zusammensetzungen und Komposit-Kolloide synthetisiert werden können. Der Lösung kann mindestens ein Additiv beigefügt sein, es kann aber auch eine Kombination von mindestens zwei Additiven, insbesondere der genannten Additive eingesetzt werden. Zur Herstellung der Komposit-Kolloide können die Additive vor oder während der Synthese der Hydridosilanlösung zugegeben werden.

Die Konzentration des Hydridosilans bzw. der Hydridosilane im Lösungsmittel kann zwischen 0 bis ≤ 100 Gew.% betragen Bei Verwendung einer Hydridosilanlösung ohne Additive wird beispielsweise eine 0,1 - 50 Gew. %ige Hydridosilankonzentration verwendet, der Rest ist Lösungsmittel. Bei Verwendung einer Hydridosilanlösung mit Additiven wird beispielsweise eine 0,01 - 50 gew.%ige Konzentration von Additiven verwendet, der Rest besteht aus Hydridosilanen und Lösungsmitteln.

Mit diesem Verfahren können Komposit-Kolloide einer Partikelgröße in einem Bereich von 0,5 nm - 10 µm hergestellt werden, insbesondere in einem Bereich von 1,5 nm - 1 µm.

Das Verfahren ermöglicht durch lange Beschallungszeiten und/oder Filterung-bzw. Trennverfahren wie beispielsweise durch Anwendung von Membranfiltern oder Zentrifugation und/oder anschließende thermische und/oder photolytische Behandlung die erfindungsgemäße Herstellung von besonderen Kolloiden mit vorteilhaften Eigenschaften wie die Bildung von Agglomeraten und Aggregaten größer als 200 nm. In einer weiteren Ausführungsform können mit dem erfindungsgemäßen Verfahren Additive oder Substanzen hergestellt werden, welche mit hydrogenierten amorphen siliciumhaltigen Beschichtungen umhüllt sind.

Dazu werden der Lösung, umfassend das Lösungsmittel und das Hydridosilan und fakultativ mindestens ein Additiv, mindestens eine Substanz zugefügt, welche durch die Einwirkung des Ultraschalls auf die Lösung mit hydrogeniertem amorphem siliciumhaltigem Material beschichtet werden soll.

Die Additive oder Substanzen, die verkapselt werden sollen, können fest und/oder flüssig und/oder gasförmig sein.

Als Additive oder als zu beschichtende und/oder einzubettende und/oder zu verkapselnde Substanzen können Nanopartikel und/oder auch Kolloide bestehend, aber nicht beschränkend, aus Au, c-Si, CdSe, CuO, Cu₂O, Cu₂S, CuS, Fe₃O₄, Fe₂O₃, FeS, FeS₂, FeSi₂, Li, LiH, SnS, ZnS, ZrS und auch Moleküle wie Alendronate, Cisplatin, Doxorubicin, Epirubicin, Fluorouracil, Idarubicin, sowie auch Verbindungen aus der Gruppe der Pentine wie 1- oder 2-Pentin oder Borane wie Diboran, Pentaboran oder Decaboran, weißer Phosphor oder auch allgemeine Verbindungen wie Phosphane oder Phosphine eingesetzt werden. Es können auch mindestens zwei Substanzen, insbesondere aus der Gruppe der genannten Additive oder Substanzen verwendet werden um damit beschichtete Gemische von Substanzen herzustellen.

Mit dem erfindungsgemäßen Verfahren können Additive oder Substanzen in Lösung direkt implosiv in eine Hülle von hydrogenierten amorphen siliciumhaltigen Kolloiden eingebettet, bzw. verkapselt werden. Dies ist mit anderen Verfahren nicht möglich, da Standard-Partikelsynthesen auf klassischen photolytischen und/oder thermolytischen Prozessen bzw. Wachstum mittels Nukleationsmechanismen beruhen.

Unter Komposit-Kolloiden werden im Sinne der Erfindung darüber hinaus auch Kolloide verstanden, die unter Verwendung von festen und/oder flüssigen und/oder gasförmigen Additiven oder Substanzen, mittels Kavitation synthetisiert sind und wo die Additive oder Substanzen mit hydrogenierten amorphen siliciumhaltigen Beschichtungen vollkommen verkapselt oder teilweise oder vollkommen mit hydrogenierten amorphen siliciumhaltigen Schichten beschichtet sind. Die Additive oder Substanzen können mit oder ohne chemische Verbindungen zum Wasserstoff und/oder Silicium im Komposit-Kolloid vorhanden sein.

Da das Verfahren ein Flüssigphasenprozess ist, der bei Raum- oder niedrigen Temperaturen und Normaldruck durchführbar ist, wird keine Hochvakuum-Technik benötigt, was einen erfindungsgemäßen Vorteil darstellt.

In einer vorteilhaften Ausführung des Verfahrens sollte die Herstellung der hydrogenierten amorphen siliciumhaltigen Kolloide vorzugsweise in einer Ar- oder N₂-Atmosphäre durchgeführt werden, wie beispielsweise in einer Glovebox mit H₂O- und O₂-Konzentrationen von typischerweise < 10 ppm.

Die Erfindung ermöglicht die direkte Einbettung und/oder Verkapselung von Substanzen, die sich innerhalb von implodierenden Mikrobläschen befinden können.

Die erfindungsgemäß erhaltenen hydrogenierten, amorphen, siliciumhaltigen Komposit-Kolloide, können beispielsweise in photovoltaischen oder photokatalytischen Anwendungen als Lichtabsorber- und/oder Dotiermaterial oder plasmonische Reflexionsgitter oder lichtstreuende Schichten, für Sensoren, Quantum-Dot Leuchtdioden, Wellenleiter, Singulett-Sauerstoff-Erzeugung, Abwasseraufbereitung, als Anodenmaterial in energiespeichernden Anwendungen, wie beispielsweise in Lithium-Ionen-, Natrium-lonen- bzw. Lithium-Sulfid-Batteriezellen verwendet werden. Die erfindungsgemäß erhaltenen mit hydrogenierten amorphen siliciumhaltigen Kolloiden bzw. Komposit-Kolloiden beschichteten bzw. verkapselten Substanzen können für in vitro oder in vivo Medikamententräger, für in vitro oder in vivo photofluoreszente oder magnetresonanztomographische bildgebende Verfahren als fluoreszenter Marker und/oder magnetresonanztomographischer Marker oder Kontrastmittel, als in vitro oder in vivo Therapeutikum, Medikamententräger und/oder Hyperthermie-Therapeutikum, oder in photodynamischer Therapie verwendet werden.

Insbesondere können die Kolloide bzw. die Komposit-Kolloide bzw. die beschichteten bzw. verkapselten Substanzen für Solarzellen, Anoden für Lithium-Ionen-Batteriezellen und als Therapeutika in bio- oder nanomedizinischem Bereich verwendet werden.

Erfindungsgemäß werden Komposit-Kolloide und mit hydrogenierten amorphen siliciumhaltigen Komposit-Kolloiden verkapselte Substanzen zur Verfügung gestellt, die dadurch gekennzeichnet sind, dass sie mit dem erfindungsgemäßen Verfahren hergestellt werden können.

In einer vorteilhaften Ausführung des Verfahrens kann nach der Beschichtung eine Sinterung und/oder Kristallisation in wasserstoffhaltiger und/oder Niederdruck-Atmosphäre durchgeführt werden.

In einer weiteren vorteilhaften Ausführung des Verfahrens kann während der Herstellung eine UV-Bestrahlung und/oder Mikrowellen-Bestrahlung und/oder aktive Kühlung vorgenommen werden.

In einer besonders bevorzugten Ausführung werden die erfindungsgemäßen Kolloide oder Komposit-Kolloide aus Trisilan unter Verwendung von deionisiertem Wasser als Lösungsmittel hergestellt. Diese so hergestellten Nanopartikel sind hydrophil und bilden stabile Dispersionen im Wasser und sind sowohl oxidiert als auch hydrogeniert.

### Spezieller Beschreibungsteil

Nachfolgend wird der Gegenstand der Erfindung anhand von Ausführungsbeispielen und Figuren näher erläutert, ohne dass der Gegenstand der Erfindung dadurch beschränkt wird.

### Ausführungsbeispiele

Es zeigen:
Figur 1 - Röntgenphotoelektronen-Spektrum an Siliciumcarbid Komposit-Kolloiden hergestellt durch Beschallung einer Trisilan-Lösung mit 2-Pentin als Additiv.
Figur 2 - Transmissionselektronenmikroskop-Aufnahme von Siliciumcarbid Komposit-Kolloiden.
Figur 3 - Rastertransmissionselektronenmikroskop-Aufnahme und Elementanalyse mittels energiedispersiver Röntgenspektroskopie von Fe₃O₄-Nanopartikeln ohne erfindungsgemäße Ultraschallbehandlung
Figur 4 - Rastertransmissionselektronenmikroskop-Aufnahme in Figur 4 (HAADF) und Elementanalyse mittels energiedispersiver Röntgenspektroskopie von Fe₃O₄-Nanopartikeln beschichtet mit einer dünnen Siliciumschicht nach erfindungsgemäßer Ultraschallbehandlung
Figur 5 - Rasterelektronenmikroskop-Aufnahme von erfindungsgemäß hergestellten siliciumhaltigen Kolloiden.
Figur 6 - Rasterelektronenmikroskop-Aufnahme eines erfindungsgemäß hergestellten siliciumhaltigen korallenartigen Kolloids nach Tempervorgang.
Figur 7 - Rasterelektronenmikroskop-Aufnahme einer hochporösen Schicht aus siliciumhaltigen Kolloiden hergestellt bei Raumtemperatur mittels Rotationsbeschichtung.
Figur 8 - Rasterelektronenmikroskop-Nahaufnahme einer hochporösen Schicht aus siliciumhaltigen Kolloiden hergestellt bei Raumtemperatur mittels Rotationsbeschichtung.
Figur 9 - Cyclovoltammetrie-Messung des ersten Lithiierungs- bzw. Delithiierungszyklus von einer porösen Dünnschicht-Anode aus erfindungsgemäß hergestellten siliciumhaltigen Komposit-Kolloiden.
Figur 10 - Cyclovoltammetrie-Messung des zweiten Lithiierungs- bzw. Delithiierungszyklus von einer porösen Dünnschicht-Anode aus erfindungsgemäß hergestellten siliciumhaltigen Komposit-Kolloiden.
Figur 11 - Galvanostatische Zyklisierung und Coulomb-Effizienz von einer porösen Dünnschicht-Anode aus erfindungsgemäß hergestellten siliciumhaltigen Komposit-Kolloiden.
Figur 12 - Fourier-Transform-Infrarot-Spektrum von erfindungsgemäßen silicium-wasserstoffhaltigen Komposit-Kolloiden hergestellt durch Beschallung einer Lösung aus Trichlorsilan anstatt Trisilan.
Figur 13 - HAADF-Aufnahme mittels STEM worauf erfindungsgemäße siliciumhaltigen Nanopartikel mit Durchmessern zwischen 50 - 200 nm dargestellt sind
Figur 14 - HAADF-Aufnahme vom Bereich der Schale eines erfindungsgemäßen Nanopartikels und im Einsatzbild oben rechts die dazugehörige ortsaufgelöste elementspezifische Verteilung von Silicium (Si) mittels energiedispersiver Röntgenspektroskopie.
Figur 15 - FTIR-Spektrum von aus Trisilan hergestellten silicium-wasserstoffhaltigen Komposit-Kolloiden unter Verwendung von deionisiertem Wasser (H₂O) als Lösungsmittel.

### Ausführungsbeispiele

### Beispiel 1 - Röntgenphotoelektronen-Spektrum an Siliciumcarbid Komposit-Kolloiden hergestellt durch Beschallung einer Trisilan-Lösung mit 2-Pentin als Additiv

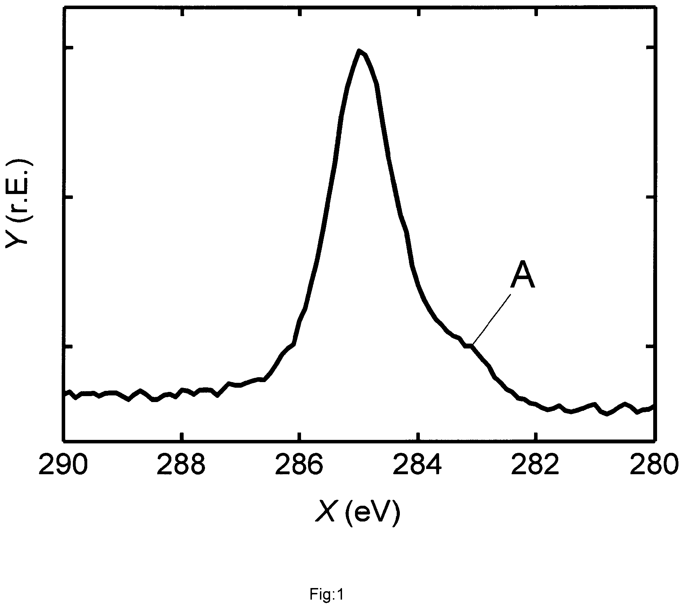

Anhand von in Figur 1 dargestellter Röntgenphotoelektronenspektroskopie (XPS) Messung ist die erfindungsgemäße Synthese von Siliciumcarbid Komposit-Kolloiden gezeigt. Das SCPS-Spektrum zeigt den C 1s Peak von Kohlenstoff. Die Abszisse *X* gibt die Bindungsenergie in (eV) an und die Ordinate *Y* gibt die XPS-Intensität in relativen Einheiten (r.E.) an. Die verwendete Cyclooctan-Lösung weist eine Trisilan Konzentration von 25 Vol% auf und als Additiv wurde 2-Pentin mit einer Konzentration von 13 Vol% verwendet. Die erfindungsgemäßen Bedingungen für die Beschallung waren wie folgt: Prozesstemperatur = 8 °C, Beschallungsdauer = 320 min, Sonotrodenamplitude = 216 µm. Die getrockneten Komposit-Kolloide wurden auf einem mit Gold beschichteten Glassubstrat vermessen. Die Komponente (A) bei ca. 283 eV in Figur 1 weist auf Silicium-Kohlenstoff Bindungen hin. Folglich kann eine Einbettung von Kohlenstoff-Atomen in siliciumhaltige Komposit-Kolloide festgestellt werden.

### Beispiel 2 - Rasterelektronenmikroskop-Aufnahme von Siliciumcarbid Komposit-Kolloiden

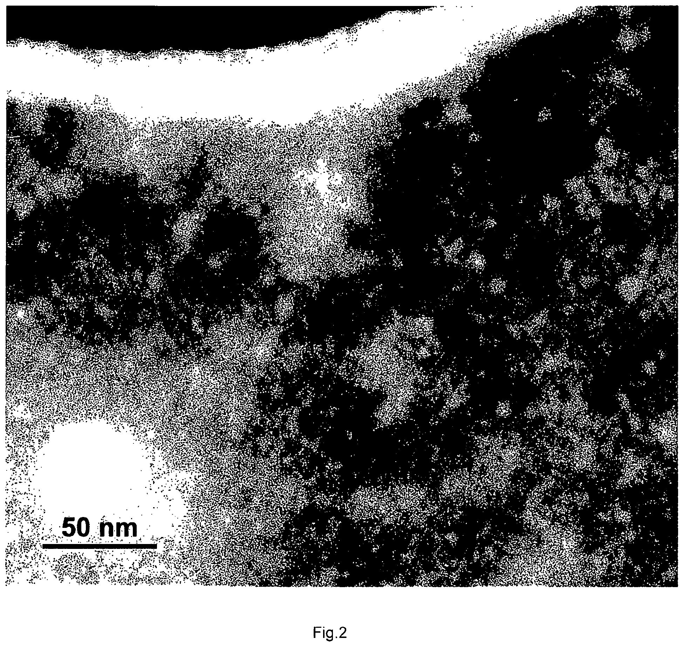

Um die Siliciumcarbid-Komposit-Kolloide aus Beispiel 1 darzustellen ist in Figur 2 eine Rasterelektronenmikroskop-Aufnahme gezeigt. Die Nanopartikel sind sphärisch und weisen Durchmesser zwischen 2 nm und 7 nm auf

### Beispiel 3 - Rastertransmissionselektronenmikroskop-Aufnahme und Elementanalyse mittels energiedispersiver Röntgenspektroskopie von Fe₃O₄-Nanopartikeln ohne erfindungsgemäße Ultraschallbehandlung

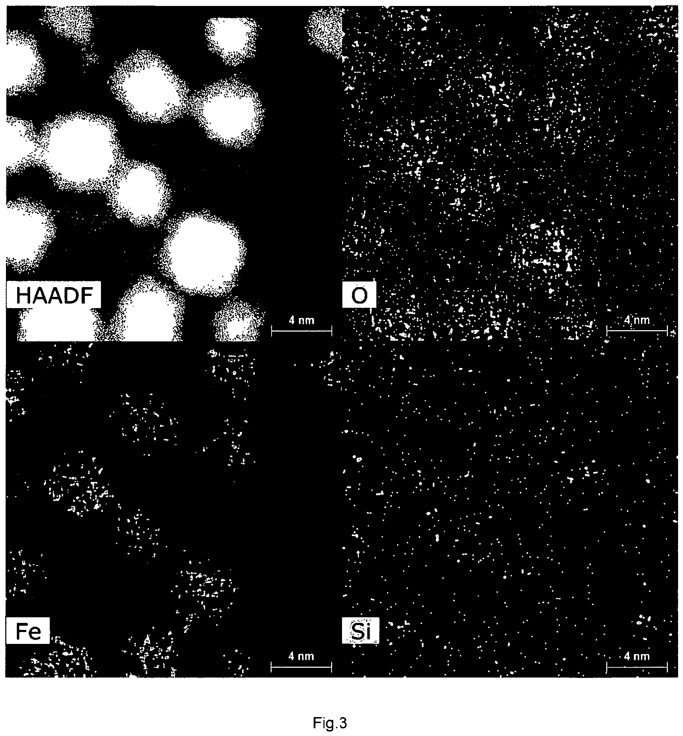

In der Rastertransmissionselektronenmikroskop-Aufnahme in Figur 3 (HAADF) oben links sind Fe₃O₄-Nanopartikel auf einem Kohlenstoffgitter gezeigt, die aus einer unbehandelten Trisilan-Cyclooctan-Lösung extrahiert wurden. Die restlichen mittels energiedispersiver Röntgenspektroskopie aufgenommenen Bilder zeigen ortsaufgelöste elementspezifische Verteilungen von Sauerstoff (O), Eisen (Fe) und Silicium (Si). Es gibt keine örtliche Korrelation zwischen der Si-Verteilung und der Position der Fe₃O₄-Nanopartikel. Folglich kann kein Rückschluss auf eine Beschichtung oder Verkapselung der Fe₃O₄-Nanopartikel mit Silicium gezogen werden.

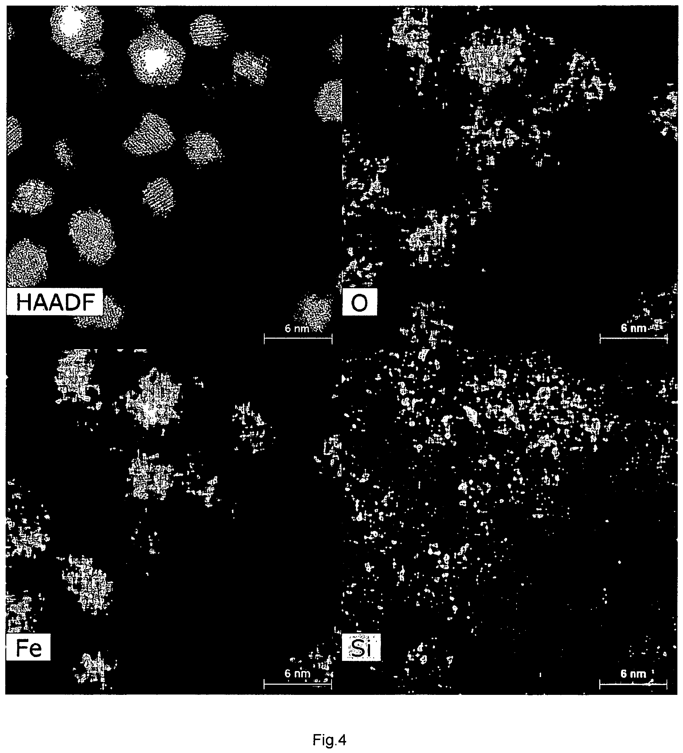

### Beispiel 4 - Rastertransmissionselektronenmikroskop-Aufnahme in Figur 4 (HAADF) und Elementanalyse mittels energiedispersiver Röntgenspektroskopie von Fe₃O₄-Nanopartikeln beschichtet mit einer dünnen Siliciumschicht nach erfindungsgemäßer Ultraschallbehandlung

In der Rastertransmissionselektronenmikroskop-Aufnahme oben links sind Fe₃O₄-Nanopartikel auf einem Kohlenstoffgitter gezeigt, die aus der Lösung vom Beispiel 3 nach 140-minütiger Beschallung mit einer Sonotrodenamplitude von 230 µm extrahiert wurden. Die restlichen mittels energiedispersiver Röntgenspektroskopie aufgenommenen Bilder zeigen ortsaufgelöste elementspezifische Verteilungen von Sauerstoff (O), Eisen (Fe) und Silicium (Si). Es ist eine örtliche Korrelation zwischen der Si-Verteilung und der Position der Fe₃O₄-Nanopartikel zu erkennen. Folglich kann eine Beschichtung oder Verkapselung der Fe₃O₄-Nanopartikel mit Silicium festgestellt werden.

### Beispiel 5 - Rasterelektronenmikroskop-Aufnahme von bei niedriger Temperatur erfindungsgemäß hergestellten siliciumhaltigen Kolloiden

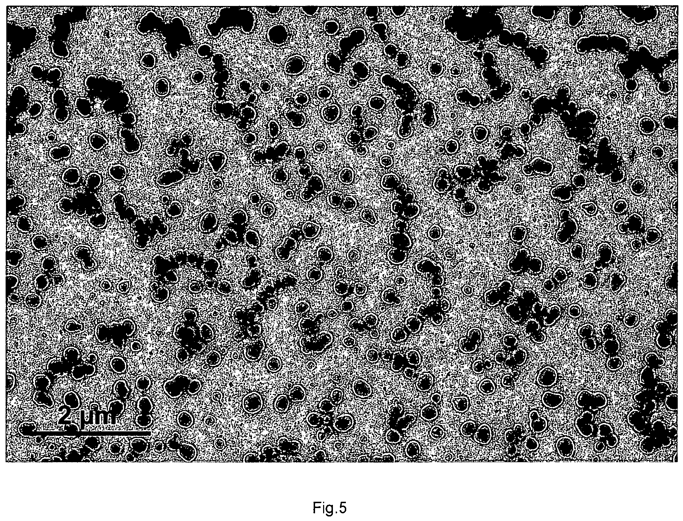

Wie in der Rasterelektronenmikroskop-Aufnahme von Figur 5 gezeigt wird, führt eine Beschallung bei einer Temperatur von ca. -3 °C und eine anschließende Filterung (0,1 µm PTFE-Spritzenvorsatzfilter) zur Herstellung von u.a. gut definierten ca. 200 nm großen siliciumhaltigen Kolloid-Agglomeraten.

### Beispiel 6 - Rasterelektronenmikroskop-Aufnahme eines erfindungsgemäß hergestellten siliciumhaltigen korallenartigen Kolloids nach Tempervorgang

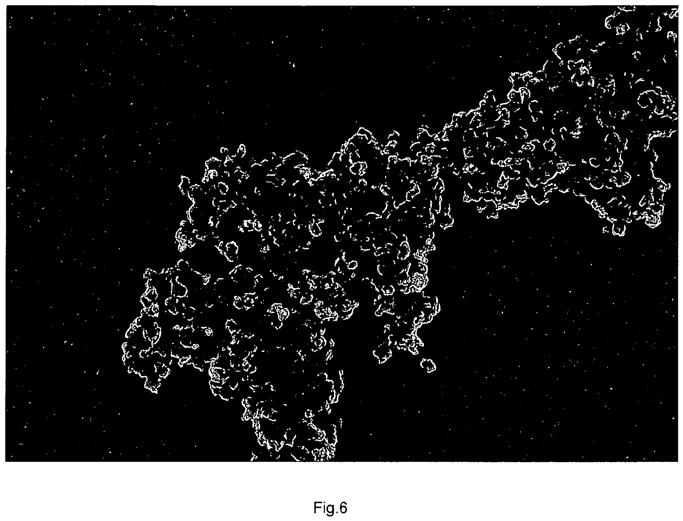

Die Rasterelektronenmikroskop-Aufnahme von Figur 6 zeigt siliciumhaltige Kolloide nach Tropfenbeschichtung und anschließender Temperung bei ca. 450 °C für 20 Minuten. Die Morphologie nimmt eine korallenartige Form an.

### Beispiel 7 - Rasterelektronenmikroskop-Aufnahme einer hochporösen Schicht aus siliciumhaltigen Kolloiden hergestellt bei Raumtemperatur mittels Rotationsbeschichtung

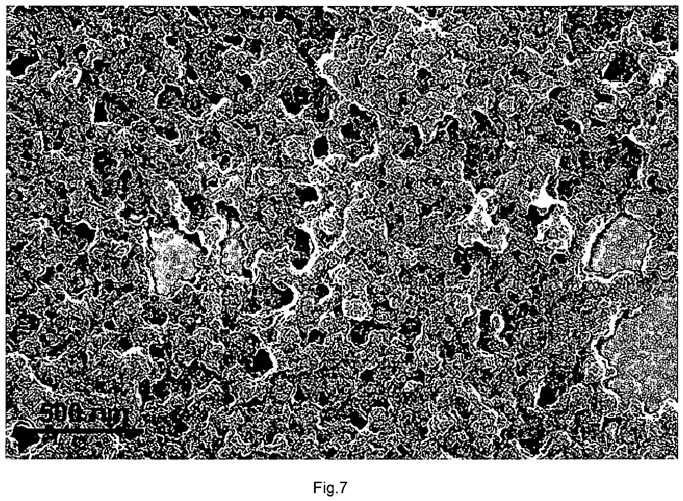

Die Rasterelektronenmikroskop-Aufnahme von Figur 7 zeigt siliciumhaltige Kolloide nach Rotationsbeschichtung bei Raumtemperatur. Die Kolloid-Schicht weist eine schwammartige Morphologie auf.

### Beispiel 8 - Rasterelektronenmikroskop-Nahaufnahme einer hochporösen Schicht aus siliciumhaltigen Kolloiden hergestellt bei Raumtemperatur mittels Rotationsbeschichtung

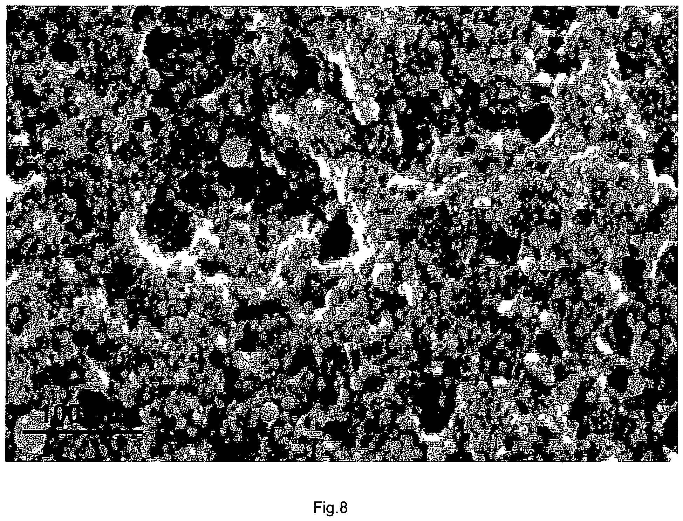

Die Rasterelektronenmikroskop-Nahaufnahme von Figur 8 zeigt die hochporöse Morphologie von siliciumhaltigen Kolloid-Dünnschichten.

### Beispiel 9 - Potentiostatische Cyclovoltammetrie-Messungen an porösen Dünnschicht-Anoden aus erfindungsgemäß hergestellten siliciumhaltigen Komposit-Kolloiden

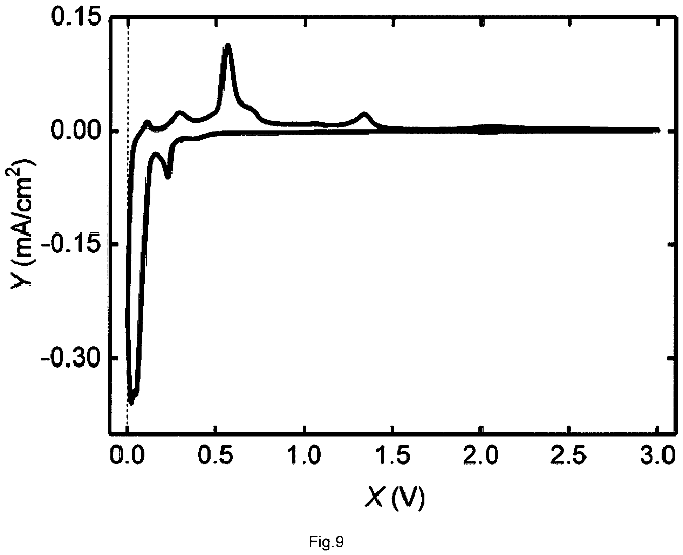

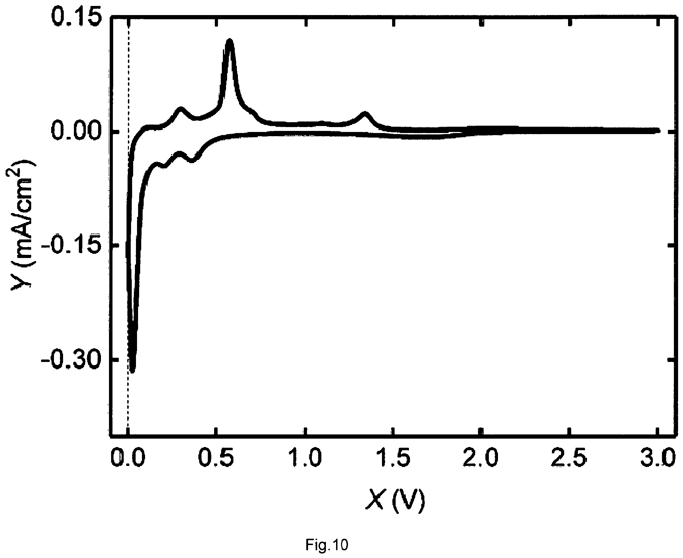

Figur 9 zeigt den ersten Lithiierungs- bzw. Delithiierungszyklus einer erfindungsgemäßen hergestellten Anode in einer Drei-Elektroden-Anordnung mit metallischem Lithium als Referenz- und Gegenelektrode. Die Abszisse *X* gibt die Spannung in (V) an und die Ordinate Y gibt die spezifische Kapazität in (mA/cm²) an. Die Anode besteht aus siliciumhaltigen Komposit-Kolloiden mit zwischen 10 - 15 at% Kohlenstoff an der Oberfläche hergestellt durch Tempern bei 300 °C für 10 Minuten. Der Elektrolyt besteht aus 1 M LiClO₄ in Propylencarbonat. Die Messungen wurden bei 1 mVls aufgenommen und die Spannung ist gegen Li/Li⁺ angegeben. Figur 10 zeigt den zweiten Lithiierungs- bzw. Delithiierungszyklus. Keinerlei Bindemittel und/oder leitfähige Zusatzstoffe wurden für die Herstellung der Anode verwendet.

### Beispiel 10 - Galvanostatische Messungen an porösen Dünnschicht-Anoden aus erfindungsgemäß hergestellten siliciumhaltigen Nanopartikeln

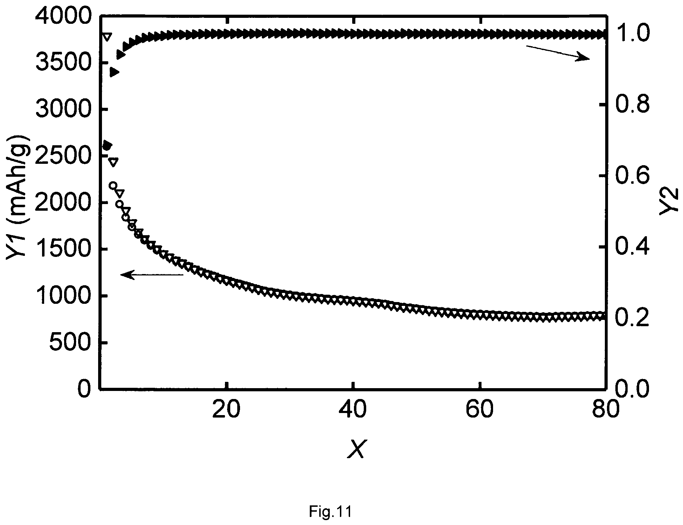

In Figur 11 sind galvanostatische Messungen an der Anode aus Beispiel 9 gezeigt. Die Abszisse X gibt die Zyklusnummer und die Ordinate *Y1* die spezifische Kapazität in (mA/cm²) an. Die Ordinate Y2 gibt die Coulomb-Effizienz an. Die Messungen wurden bei einer Auf- bzw. Entladerate von 0.5 C (104 2 µA) mit Grenzspannungen von 0 V und 3 V aufgenommen. Die Coulomb-Effizienz beträgt mehr als 99.7% nach 80 Zyklen.

### Beispiel 11 - Fourier-Transformiertes-Infrarot-Spektrum von erfindungsgemäßen silicium-wasserstoffhaltigen Komposit-Kolloiden hergestellt durch Beschallung einer Lösung aus Trichlorsilan anstatt Trisilan.

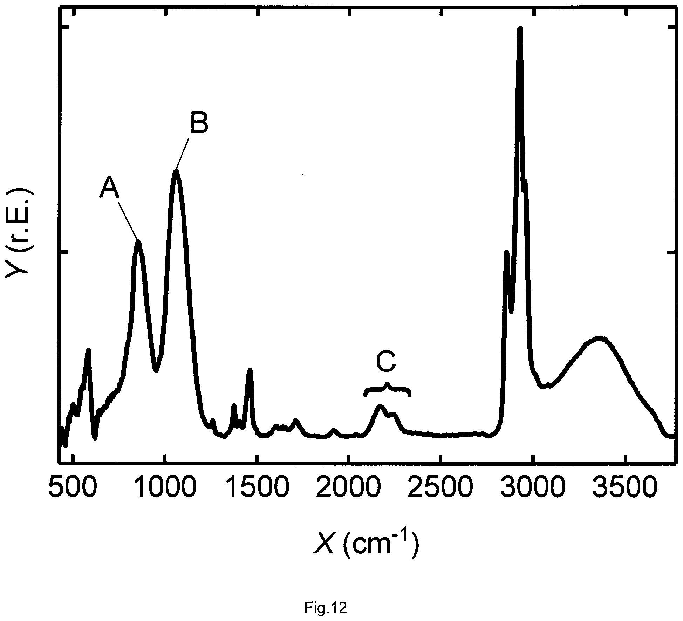

Figur 12 zeigt ein Fourier-Transformiertes-Infrarot (FTIR)-Spektrum von aus Trichlorsilan hergestellten silicium-wasserstoffhaltigen Komposit-Kolloiden, Die Komposit-Kolloide wurden auf ein Silicium-Wafer aufgebracht, getrocknet und anschließend bei Raumtemperatur vermessen. Die Abszisse *X* gibt die Wellenzahl in (cm⁻¹) und die Ordinate *Y* die FTIR-Signalintensität in relativen Einheiten (r.E.) an. Die Mode (A) deutet auf Si-H_{1,2} Biege- bzw. Scherenschwingen, die Mode (B) deutet auf die Si-O-Si Streckschwingen, und die Moden (C) deuten auf Si-H_{1.2} Streckschwingen hin. Diese Moden sind charakteristisch für amorphe silicium-wasserstoffhaltige Festkörper-Kolloide.

### Beispiel 12 - Rastertransmissionselektronenmikroskop-Aufnahmen (STEM) und Elementanalyse mittels energiedispersiver Röntgenspektroskopie von in wässrigem Milieu synthetisierten siliciumhaltigen Nanopartikeln

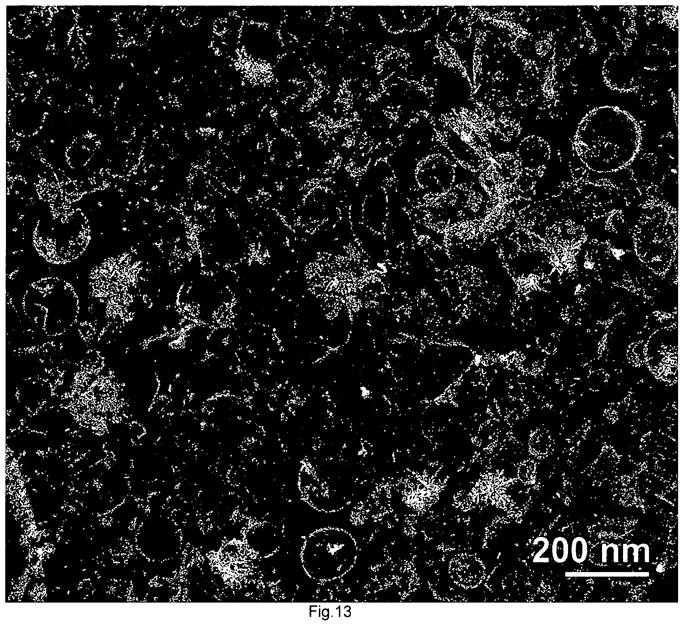

Figur 13 zeigt eine HAADF-Aufnahme mittels STEM, worauf siliciumhaltige Kolloide mit Durchmessern zwischen 50 - 200 nm dargestellt sind. Die Beschaffenheit dieser erfindungsgemäßen Kolloide ist dadurch gekennzeichnet, dass sie, wie Figur 13 zeigt, bevorzugt eine sphärische Geometrie aufweisen, hohl sind und eine Schale aufweisen, die bevorzugt eine Dicke zwischen 3 - 10 nm aufweist. Diese erfindungsgemäße Architektur der Kolloide ist durch das erfindungsgemäße Verfahren unter Verwendung von Trisilan-Precursor (Si₃H₈) und deionisiertem Wasser (H₂O) als Lösungsmittel und eine besondere Wahl aus Arbeitszyklus (60 - 70 %), Amplitude (190 - 220 µm) Trisilan-Konzentration (5 - 15 vol%) und Temperatur (5 - 10 °C) ermöglicht. Unter der Bezeichnung Arbeitszyklus in Prozent- Angabe ist der aktive Zeitraum einer Sonotrodelder Ultraschallquelle an der gesamten Beschallungszeit. So bedeutet ein Arbeitszyklus von 60%, dass die Sonotrode für 600 Millisekunden eingeschaltet und für 400 Millisekunden ausgeschaltet ist. Die Angabe zur Amplitude bedeutet, die Sonotrodenamplitude, d. h. die maximale räumliche Hin-und Her-Bewegung der Sonotrodenspitze.

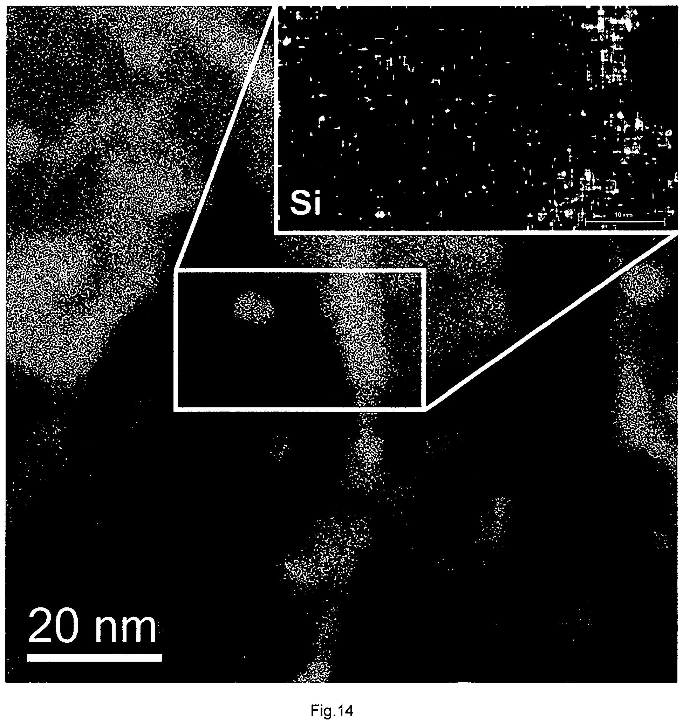

Figur 14 zeigt eine HAADF-Aufnahme vom Bereich der Schale eines erfindungsgemäßen Kolloids und im Einsatzbild oben rechts die dazugehörige ortsaufgelöste elementspezifische Verteilung mittels energiedispersiver Röntgenspektroskopie (= EDX) von Silicium (Si). Die Elementanalyse zeigt die siliciumhaltige Zusammensetzung der Kolloide.

### Beispiel 13 - Fourier-Transformiertes-Infrarot-Spektrum (FTIR) von erfindungsgemäßen hydrogenierte siliciumhaltigen Komposit-Kolloiden hergestellt durch Beschallung einer wässrigen Trisilanlösung

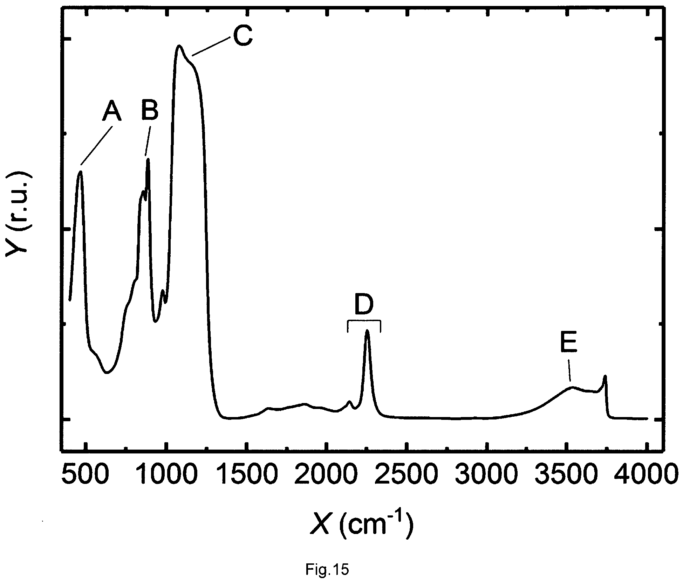

Figur 15 zeigt ein FTIR-Spektrum von aus Trisilan hergestellten silicium-wasserstoffhaltigen Komposit-Kolloiden unter Verwendung von deionisiertem Wasser (H₂O) als Lösungsmittel. Die Komposit-Kolloide wurden von einer stabilen Dispersion entnommen und auf einen Silicium-Wafer aufgebrachten, getrocknet und anschließend bei Raumtemperatur vermessen. Die Abszisse X gibt die Wellenzahl in (cm⁻¹) und die Ordinate Y die Signalintensität in relativen Einheiten (r.E.) an. Die Mode (A) deutet auf die SiO Biegeschwingung, die Mode (B) deutet auf die SiHx Biegeschwingung, die Moden (C) deuten auf die Si-O-Si StreckSchwingung, die Moden (D) deuten auf O_{y}Si-Hₓ Streckschwingungen und die Mode (E) deuten auf H₂O Streckschwingung hin. Diese Moden sind charakteristisch für amorphe silicium-wasserstoffhaltige Kolloide. Insbesondere ist aufgrund von Moden (C) und (D) zurückzuschließen, dass diese Komposit-Kolloide sowohl oxidiert (sauerstoffhaltig) als auch hydrogeniert (wasserstoffhaltig) sind.

## Patentansprüche

1. Hydrogenierte amorphe siliciumhaltige Kolloide oder Komposit-Kolloide **dadurch gekennzeichnet,**
**dass** sie eine siliciumhaltige Schale aufweisen, die die hohlen Kolloide oder Komposit-Kolloide umgeben, wobei diese eine sphärische Geometrie aufweisen, wobei die siliciumhaltigen Komposit-Kolloide in rasterelektronenmikroskopischen Aufnahmen eine sphärische Geometrie und einen Durchmesser zwischen 2 nm und 7 nm aufweisen und wobei die siliciumhaltigen Kolloide in rastertransmissionselektronenmikroskopischen Aufnahmen eine sphärische Geometrie mit einem Hohlraum und einem Durchmesser zwischen 50 bis 200 nm aufweisen, wobei der Hohlraum von einer Schale mit einer Dicke zwischen 3 bis 10 nm umgeben ist.

2. Verfahren zur Herstellung von hydrogenierten amorphen siliciumhaltigen Kolloiden oder Komposit-Kolloiden nach Anspruch 1 sowie zur Verkapselung von Substanzen mit diesen hydrogenierten amorphen siliciumhaltigen Komposit-Kolloiden, sowie hydrogenierte amorphe siliciumhaltige Komposit-Kolloide und mit hydrogenierten amorphen siliciumhaltigen Komposit-Kolloiden verkapselte Substanzen, **dadurch gekennzeichnet,**
**dass** zur Herstellung der Komposit-Kolloide eine Trisilan-Cyclooctan Lösung mit einem Additiv, wobei die Trisilan Konzentration 25 Vol% und die Additiv Konzentration 13 Vol% beträgt, mit Kavitation durch eine Sonotrode beaufschlagt wird, und die Beschallung durch die Sonotrode bei einer Prozesstemperatur von 8°C, einer Beschallungsdauer von 320 min und einer Sonotrodenamplitude von 216 µm durchgeführt wird und zur Herstellung der Kolloide ein Trisilan- Precursor (Si₃H₈) in Wasser als Lösungsmittel mit Kavitation durch eine Sonotrode beaufschlagt wird, mit einem Arbeitszyklus von 60-70%, einer Sonotrodenamplitude zwischen 190-220 µm, einer Trisilankonzentration zwischen 5 bis 15 Vol%, bei einer Prozesstemperatur zwischen 5-10°C.

3. Verfahren nach einem der vorhergehenden Ansprüche,
**dadurch gekennzeichnet,**
**dass** als Additiv Substanzen eingesetzt werden, die fest, flüssig oder gasförmig sind.

4. Verfahren nach einem der vorhergehenden Ansprüche,
**dadurch gekennzeichnet,**
**dass** als Additiv oder als Substanz mindestens eine Komponente aus der Gruppe Nanopartikel, vorzugsweise aus Au, c-Si, CdSe, CuO, Cu₂O, Cu₂S, CuS, Li, LiH, Fe₃O₄, Fe₂O₃, FeS, FeS₂, FeSi₂, SnS, ZnS, ZrS, Moleküle, vorzugsweise Alendronate, Cisplatin, Doxorubicin, Epirubicin, Fluorouracil, Idarubicin, Verbindungen aus der Gruppe der Pentine, vorzugsweise 1- oder 2-Pentin oder lithiumhaltige Verbindungen oder Dotierstoffe, wie borhaltige und/oder phosphorhaltige Verbindungen, oder Borane, vorzugsweise Diboran, Pentaboran oder Decaboran, weißer Phosphor oder allgemeine Verbindungen wie Phosphane oder Phosphine eingesetzt wird.

5. Verfahren nach einem der vorhergehenden Ansprüche,
**dadurch gekennzeichnet,**
**dass** mindestens eine Substanz zugefügt wird, welche bedingt durch die vorhergehenden Verfahrensschritte mit einer siliciumhaltigen Schicht verkapselt bzw. beschichtet bzw. in eine siliciumhaltige Schicht eingebettet wird.

6. Verfahren nach einem der vorhergehenden Ansprüche,
**dadurch gekennzeichnet,**
**dass** nach der Beschichtung eine Sinterung und/oder Kristallisation in wasserstoffhaltiger und/oder Niederdruck-Atmosphäre durchgeführt wird.

7. Verfahren nach einem der vorhergehenden Ansprüche,
**dadurch gekennzeichnet,**
**dass** während der Herstellung eine UV-Bestrahlung und/oder Mikrowellen-Bestrahlung und/oder aktive Kühlung vorgenommen wird.

8. Verwendung von einem hydrogenierten amorphen siliciumhaltigen Komposit-Kolloid nach Anspruch 1 zur Anwendung als Anodenmaterial in einem energiespeichernden Bauelement.

9. Verwendung von einem hydrogenierten amorphen siliciumhaltigen Komposit-Kolloid nach Anspruch 1 zur Anwendung als Therapeutikum, Medikamententräger und/oder fluoreszenter Marker und/oder magnetresonanztomographischer Marker oder Kontrastmittel und/oder Hyperthermie-Therapeutikum.

## Claims

1. A hydrogenated amorphous silicon-containing colloids or composite colloids, **characterized in that** they have a silicon-containing shell which surrounds the hollow colloids or composite colloids, which have a spherical geometry, wherein the silicon-containing composite colloids have a spherical geometry and a diameter of between 2 nm and 7 nm in scanning electron micrographs and wherein the silicon-containing colloids have a spherical geometry with a cavity and a diameter of between 50 to 200 nm in scanning transmission electron micrographs, the cavity being surrounded by a shell with a thickness of between 3 to 10 nm.

2. A method for producing hydrogenated amorphous silicon-containing colloids or composite colloids according to claim 1 and for encapsulating substances with these hydrogenated amorphous silicon-containing composite colloids, as well as hydrogenated amorphous silicon-containing composite colloids and substances encapsulated with hydrogenated amorphous silicon-containing composite colloids,
**characterized in that**, in order to produce the composite colloids, a trisilane-cyclooctane solution with an additive, the trisilane concentration being 25 vol% and the additive concentration being 13 vol%, is subjected to cavitation by a sonotrode, and the sonication by the sonotrode is carried out at a process temperature of 8 °C, a sonication time of 320 min, and a sonotrode amplitude of 216 m, and in order to produce the colloids, a trisilane precursor (Si3H8) in water as a solvent is subjected to cavitation by a sonotrode, with a duty cycle of 60-70%, a sonotrode amplitude of between 190-220 m, a trisilane concentration of between 5 to 15 vol%, at a process temperature of between 5-10 °C.

3. The method according to any one of the preceding claims, **characterized in that** substances which are solid, liquid, or gaseous are used as additives.

4. The method according to any one of the preceding claims, **characterized in that** at least one component from the group of nanoparticles, preferably from Au, c-Si, CdSe, CuO, Cu20, Cu2S, CuS, Li, LiH, Fe₃O₄, Fe203, FeS, FeS2, FeSi2, SnS, ZnS, ZrS, molecules, preferably alendronate, cisplatin, doxorubicin, epirubicin, fluorouracil, idarubicin, compounds from the group of pentines, preferably 1- or 2-pentine or lithium-containing compounds or dopants, such as boron-containing and/or phosphorus-containing compounds, or boranes, preferably diborane, pentaborane, or decaborane, white phosphorus, or general compounds such as phosphanes or phosphines is used as an additive or as a substance.

5. The method according to any one of the preceding claims, **characterized in that** at least one substance is added which, as a result of the preceding method steps, is encapsulated or coated with a silicon-containing layer or embedded in a silicon-containing layer.

6. The method according to any one of the preceding claims, **characterized in that**, after the coating, sintering and/or crystallization is carried out in a hydrogen-containing and/or low-pressure atmosphere.

7. The method according to any one of the preceding claims, **characterized in that** UV irradiation and/or microwave irradiation and/or active cooling is carried out during production.

8. A use of a hydrogenated amorphous silicon-containing composite colloid according to claim 1 for application as an anode material in an energy-storing component.

9. The use of a hydrogenated amorphous silicon-containing composite colloid according to claim 1 for application as a therapeutic agent, drug carrier and/or fluorescent marker and/or magnetic resonance imaging marker or contrast agent and/or as a therapeutic agent for hyperthermia.

## Revendications

1. Colloïdes ou colloïdes composites amorphes hydrogénés contenant du silicium,
**caractérisé**
**en ce qu'**ils ont d'une coque contenant du silicium, qui entoure les colloïdes creux ou les colloïdes composites, ceux-ci ayant une géométrie sphérique, les colloïdes composites contenant du silicium ayant, dans des enregistrements au microscope électronique à balayage, une géométrie sphérique et un diamètre compris entre 2 nm et 7 nm
et les colloïdes contenant du silicium ayant, dans des enregistrements au microscope électronique de transmission à balayage, une géométrie sphérique ayant une cavité et un diamètre compris entre 50 et 200 nm, la cavité étant entourée d'une coque d'une épaisseur comprise entre 3 et 10 nm.

2. Procédé de préparation de colloïdes ou de colloïdes composites amorphes hydrogénés contenant du silicium suivant la revendication 1, ainsi pour l'encapsulation de substances par ces colloïdes composites amorphes hydrogénés contenant du silicium, ainsi que de colloïdes composites amorphes hydrogénés contenant du silicium et de substances encapsulées par des colloïdes composites amorphes hydrogénés contenant du silicium, **caractérisé en ce que**, pour la préparation des colloïdes composites, on admet avec cavitation dans une sonotrode une solution de trisilane-cyclooctane avec un additif, la concentration en trisilane étant de 25% en volume et la concentration de l'additif de 13% en volume et on effectue la sonorisation par la sonotrode à une température opératoire de 8°C, avec une durée de sonorisation de 320 min et une amplitude de sonotrode de 216 µm et, pour la préparation des colloïdes, on admet avec cavitation dans une sonotrode un précurseur de trisilane (Si₃H₈) dans de l'eau comme solvant et on effectue la sonorisation avec un cycle de travail de 60-70%, une amplitude de sonotrode comprise entre 190-220 µm, une concentration en trisilane comprise entre 5 à 15% en volume à une température opératoire comprise entre 5-10°C.

3. Procédé suivant l'une des revendications précédentes, **caractérisé**
**en ce que** l'on utilise comme additif des substances, qui sont solides, liquides ou gazeuses.

4. Procédé suivant l'une des revendications précédentes,
**caractérisé**
**en ce que** l'on utilise comme additif ou comme substance au moins un constituant choisi dans le groupe des nanoparticules, de préférence en Au, c-Si, CdSe, CuO, Cu₂O, Cu₂S, CuS, Li, LiH, Fe₃O₄, Fe₂O₃, FeS, FeS₂, FeSi₂, SnS, ZnS, ZrS, des molécules, de préférence des alendronates, la cisplatine, la doxorubicine, l'épirubicine, le fluorouracil, l'idarubicine, des composés du groupe de la pentine, de préférence de la 1- ou 2- pentine ou des composés lithiés ou des substances de dopage comme des composés borés et/ou phosphorés ou des boranes, de préférence du diborane, du pentaborane ou du décaborane, du phosphore blanc ou des composés généraux comme des phosphanes ou des phosphines.

5. Procédé suivant l'une des revendications précédentes,
**caractérisé**
**en ce que** l'on ajoute au moins une substance, qui, en raison des stades de procédé précédents, est encapsulée ou revêtue d'une couche contenant du silicium ou est enrobée dans une couche contenant du silicium.

6. Procédé suivant l'une des revendications précédentes,
**caractérisé**
**en ce qu'**après le revêtement, on effectue un frittage et/ou une cristallisation dans une atmosphère contenant de l'hydrogène et/ou sous basse pression.

7. Procédé suivant l'une des revendications précédentes,
**caractérisé**
**en ce que** pendant la préparation, on effectue une exposition au rayonnement UV et/ou une exposition au rayonnement de micro-ondes et/ou un refroidissement actif.

8. Utilisation d'un colloïde composite amorphe hydrogéné contenant du silicium suivant la revendication 1, pour l'utilisation comme matière d'anode dans un composant accumulant de l'énergie.

9. Utilisation d'un colloïde composite amorphe hydrogéné contenant du silicium suivant la revendication 1, pour l'utilisation comme agent thérapeutique, comme substance porteuse de médicament et/ou comme marqueur fluorescent et/ou comme marqueur en tomodensitométrie par résonance magnétique ou comme agent de contraste et/ou comme agent thérapeutique d'hyperthermie.
